(19) **Europäisches Patentamt / European Patent Office / Office européen des brevets**

(11) **EP 2 872 038 B1**

(12) **EUROPEAN PATENT SPECIFICATION**

(45) Date of publication and mention of the grant of the patent:
**28.09.2016 Bulletin 2016/39**

(21) Application number: **13762273.4**

(22) Date of filing: **08.07.2013**

(51) Int Cl.:
*A61B 5/0492* (2006.01)   *A61B 8/00* (2006.01)

(86) International application number:
**PCT/IB2013/055578**

(87) International publication number:
**WO 2014/009868 (16.01.2014 Gazette 2014/03)**

(54) **DEVICE FOR THE JOINT ACQUISITION OF AT LEAST ONE SURFACE ELECTROMYOGRAPHIC SIGNAL AND ONE ECHOGRAPHIC IMAGE OF A SAME PORTION OF A MUSCLE OF A LIVING BEING, IN PARTICULAR FOR NON-INVASIVE APPLICATIONS, AND MANUFACTURING METHOD**

VORRICHTUNG ZUR GEMEINSAMEN ERFASSUNG VON MINDESTENS EINEM ELEKTROMYOGRAFISCHEN OBERFLÄCHENSIGNAL UND EINEM ECHOGRAFISCHEN BILD DESSELBEN MUSKELTEILS EINES LEBEWESENS, INSBESONDERE FÜR NICHT-INVASIVE ANWENDUNGEN, UND HERSTELLUNGSVERFAHREN

DISPOSITIF POUR L'ACQUISITION COMMUNE D'AU MOINS UN SIGNAL ÉLECTROMYOGRAPHIQUE DE SURFACE ET D'UNE IMAGE ÉCHOGRAPHIQUE D'UNE MÊME PARTIE D'UN MUSCLE D'UN ÊTRE VIVANT, EN PARTICULIER POUR DES APPLICATIONS NON INVASIVES, ET PROCÉDÉ DE FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.07.2012 IT TO20120613**

(43) Date of publication of application:
**20.05.2015 Bulletin 2015/21**

(73) Proprietor: **Politecnico Di Torino**
**10129 Torino (TO) (IT)**

(72) Inventors:
• **BOTTER, Alberto**
  **13856 Vigliano Biellese (BI) (IT)**
• **MERLETTI, Roberto**
  **10124 Torino (TO) (IT)**

(74) Representative: **Camolese, Marco et al**
**Metroconsult S.r.l.**
**Via Sestriere 100**
**10060 None (TO) (IT)**

(56) References cited:
**WO-A1-92/08407**   **WO-A1-98/51215**
**WO-A1-2009/089280**   **WO-A1-2011/061338**

EP 2 872 038 B1

## Description

The present invention relates to a device for the joint acquisition of at least one surface electromyographic signal (sEMG) and one echographic image of a same portion of a muscle of a living being, in particular for non-invasive applications, in accordance with the preamble of claim 1.

The present invention further relates to a method for manufacturing said device.

It is known in the art that electromyography (EMG) is the representation of the electric signals generated by the contraction of a muscle.

Invasive or non-invasive techniques may be used in order to record an electromyographic signal (EMG).

Invasive techniques require the use of intramuscular needles and/or wires, through which one can get information about specific and spatially-concentrated muscle areas, so as to be able to observe the activity of a few motor units or tens of motor units. Instead, non-invasive techniques utilize surface electrodes to be positioned on an individual's skin, said surface electrodes allowing to obtain more general and global information, while at the same time detecting the activity of hundreds of motor units.

This type of surface electromyography (sEMG) allows extracting information about:

- the instants of activation of the muscle,
- the amplitude of the surface electromyographic signal (sEMG), said amplitude being correlated to the number of motor units recruited,
- the spectral characteristics of the signal, which vary as a function of muscular recruitment and fatigue.

Consequently, surface electromyography (sEMG) is a non-invasive technique which allows studying the phenomena at the basis of muscle contraction through the analysis of the electric signal generated during the contraction.

The electric potentials detected on the skin provide a surface representation of the potentials generated at muscular fibre level; for this reason, their properties depend on the geometric characteristics of the fibres, such as those relating to the position and orientation thereof with respect to the signal detection system.

During a muscle contraction, the position and orientation of the fibres with respect to the signal detection system may vary significantly, thus modifying the electromyographic diagram (EMG) accordingly. For this reason, many laboratory studies are based on a constant-force isometric contraction paradigm, which allows to conjecture the stationariness of the muscular architecture. However, in most applicative studies this paradigm is not applicable because it is not representative of the "physiological" muscle contraction and because the electromyographic signal (EMG) must be analyzed in dynamic conditions.

It is nonetheless important to observe that the muscular architecture may change significantly, for different contraction levels, also in strictly isometric conditions.

For these reasons, it is important to integrate the information of the electromyographic signal (EMG) with information about the anatomical characteristics of the signal source; such characteristics can be observed by using ultrasounds and the corresponding ultrasonographic images.

Said ultrasonographic images, in fact, make it possible to study the architecture of the muscle *in vivo* and to observe any geometric variations thereof during its contraction.

For example, one interesting field of application of the joint analysis of echographic and surface electromyographic (sEMG) images is the study of the erect posture. In particular, it might be useful to be able to correlate the variations in the architecture of the surae triceps to the EMG electromyographic activation of muscular compartments in response to postural oscillations.

Consequently, a joint study of the surface electromyographic signal (sEMG) with at least one echographic image would turn out to be very useful to widen one's knowledge in this field.

Other application areas relating to the acquisition of electromyographic signals on the skin in conjunction with echographic images may find significant applications in sports medicine, ergonomics, and clinical neurophysiology. In these fields, in fact, many applications are possible which concern the sphere of neuromuscular system research: from basic studies on muscular electrophysiology to studies on the mechanisms that regulate postural control.

Possible clinical applications may concern the screening and diagnosis of myopathies and neuropathies. For example, a particularly interesting clinical application may relate to the early screening of amyotrophic lateral sclerosis (ALS), in that the joint utilization of ultrasound and surface electromyography (sEMG) may significantly increase the diagnostic sensitivity of said sclerosis.

A few publications have discussed the joint acquisition of a surface electromyographic signal (sEMG) and echographic images. The following publications can be referred to by way of example:

- De Oliveira L.F., Menegaldo L.L. "Individual-specific muscle maximum force estimation using ultrasound for ankle joint torque prediction using an EMG-driven Hill-type model". J. Biomech. 2010 Oct 19;43(14):2816-21;
- Rudroff T., Staudenmann D., Enoka R. M. "Electromyographic measures of muscle activation and changes in muscle architecture of human elbow flexors during fatiguing contractions". J. Appl. Physiol. 2008 Jun;104(6):1720-6.

- Mademli L, Arampatzis A. "Mechanical and morphological properties of the triceps surae muscle-tendon unit in old and young adults and their interaction with a submaximal fatiguing contraction". J Electromyogr. Kinesiol. 2008 Feb;18(1):89-98.
- Loram ID, Maganaris CN, Lakie M. "Human postural sway results from frequent, ballistic bias impulses by soleus and gastrocnemius". J. Physiol. 2005 Apr. 1;564(Pt 1):295-311.
- Hodges PW, Pengel LH, Herbert RD, Gandevia SC. "Measurement of muscle contraction with ultrasound imaging". Muscle Nerve. 2003 Jun;27(6):682-92.

It must be pointed out that in all of the above-mentioned publications the surface electromyographic signal (sEMG) and the echographic images have been acquired at distinct moments or from different muscle portions; as a consequence, the signals thus acquired and possibly recorded cannot be considered to be representative of a muscular contraction of one well-determined and precise muscle portion.

In particular, in the cases described in the above-mentioned publications, the echographic images and the surface electromyographic signals (sEMG) have been recorded at distinct moments or from different muscle portions because, in order to carry out the echographic analysis and the surface electromyographic analysis on the same muscle portion, it would have been necessary to position the echographic probe over the electrodes for detecting said surface electromyographic signals (sEMG). In the cases described in the above-mentioned publications, after positioning of the echographic probe over known electrodes for detecting said surface electromyographic signals, the echographic image of the muscle tissue would have turned out deformed by the high acoustic impedance of the materials composing the known electrodes, while the gel used as an interface between the echographic probe and the skin would have caused short circuits between the known electrodes for detecting the surface electromyographic signal (sEMG).

Document WO2009089280 relates to a device for acquiring at least one electromyographic signal and at least one echographic image of a living being; in particular, according to said document the device is used in an invasive manner, particularly in order to obtain an image of the prostate of a human being.

It must be pointed out that, for invasive applications, the problem of the interface between the ultrasonographic probe and the tissue is less important than in non-invasive applications, since in invasive applications the interface is mediated by the mucosa, which improves the interface between the probe and the tissue.

However, no mucosa is present on the surface of the skin of a living being, and therefore it cannot improve the interface between the probe and the tissue. It is thus apparent that the teachings of document WO2009089280 cannot be adopted for non-invasive applications in order to obtain a device for the joint acquisition of at least one surface electromyographic signal (sEMG) and at least one echographic image of a same portion of a muscle of a living being.

Document WO92/08407 discloses an apparatus for coupling a compression wave transducer to the surface of a patient, said apparatus further comprising electrical contacts including electrical pads for picking up electrical signals from the same region of the patient.

It is the main object of the present invention to overcome the drawbacks of the prior art by providing a device which allows to acquire at least one surface electromyographic signal (sEMG) in conjunction with at least one echographic image of a same portion of a muscle of a living being, in particular for non-invasive applications.

It is a further main object of the present invention to design said device in such a way that the echographic probe can be positioned over the electrodes for detecting the electromyographic signals in a manner such that said echographic probe will not be adversely affected by the presence of said electrodes.

In particular, it is one object of the present invention to design said device in such a way that the echographic image will not be deformed by the acoustic impedance of the materials used for manufacturing the device itself or those used for manufacturing the electrodes for detecting said electromyographic signals.

It is another main object of the present invention to design said device in such a way that no short circuit will occur between the elements that make up the device, in particular between the electrodes for detecting the surface electromyographic signal (sEMG).

Yet another main object of the present invention is to provide a device which is particularly suited to non-invasive applications.

Said objects are achieved by the present invention through a device for the joint acquisition of at least one surface electromyographic signal (sEMG) and at least one echographic image of a same portion of a muscle of a living being, in particular for non-invasive applications, and through a corresponding method for manufacturing said device, which incorporate the features set out in the appended claims, which are an integral part of the present description.

Further objects, features and advantages of the present invention will become apparent from the following detailed description and from the annexed drawings, which are supplied by way of non-limiting example, wherein:

- Figs. 1a and 1b respectively show a perspective view and a sectional view of a portion of the device for acquiring at least one surface electromyographic signal (sEMG), in particular for non-invasive applications, in accordance with the present invention;
- Fig. 2 shows a sectional view of a second embodiment of the device of Fig. 1;
- Figures 3a to 4d show different steps of a method for manufacturing the device depicted in Figures 1 and 2.

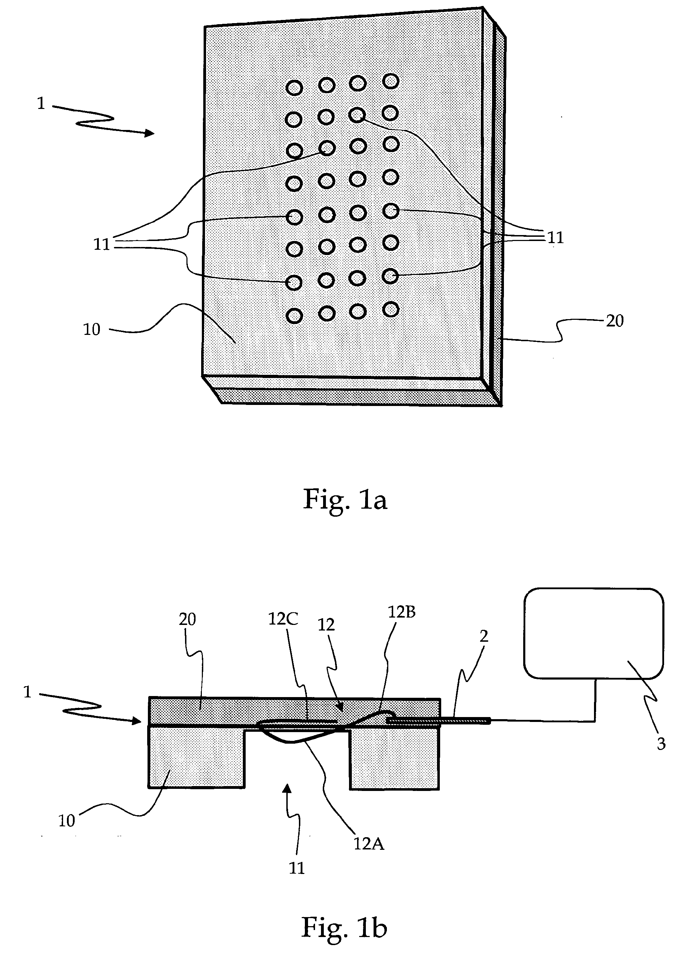

Referring now to the annexed drawings, in Figs. 1a and 1b reference numeral 1 designates as a whole a device for acquiring at least one surface electromyographic signal (sEMG) of a muscle of a living being, in particular for non-invasive applications.

In accordance with the present invention, said device 1 comprises a first layer 10 consisting of cross-linked polymers, said first layer 10 comprising at least one cavity 11 adapted to house at least one first portion 12A of an electrode 12 for acquiring said at least one surface electromyographic signal (sEMG).

Preferably, said first layer 10 is so designed as to be adapted to adhere to the skin of said living being, so as to reduce the presence of any air bubbles.

Furthermore, in accordance with the present invention said first layer 10 is transparent to ultrasounds, so as to allow obtaining an acoustic impedance adaptation such that a joint acquisition of said at least one surface electromyographic signal and of said at least one echographic image of a same muscle portion can be obtained.

Said first layer 10 consisting of cross-linked polymers is made of rubber, in particular low-density rubber; in particular, said first rubber layer 10 has a base mass/catalyst concentration (or mixing ratio) substantially equivalent to a ratio of 1 to 3.

In substance, said first layer 10 has a base mass/catalyst concentration substantially suitable for satisfying the following relation (A):

$$base\ mass : catalyst = 1 : 3$$

Preferably, said base mass and said catalyst consist of the material known as Elite Double 8®, available from company Zhermack S.p.A.

According to the present invention, said electrode 12 is of the wire type, in particular being made of metallic material; preferably, the thickness of said wire electrode 12 is comprised between 20 μm and 150 μm, in particular substantially equal to 50 μm.

In a preferred embodiment, said wire electrode 12 is made of chlorinated silver, so as to optimize the interface between the electrode 12 and a conductive paste (not shown in the drawings) housed in said cavity 11. It is however clear that the wire electrode 12 may also be made of different types of metal.

As can be seen especially in Fig. 1b, said electrode 12 comprises a second portion 12B adapted to be connected to a printed circuit board 2, said printed circuit board 2 allowing the electrode 12 to be connected to a processing and/or display system 3. For example, said processing and/or display system 3 (diagrammatically shown in Fig. 1b) may comprise a processor associated with a screen to process and display the signals coming from said electrode 12.

In addition, the electrode 12 comprises a third portion 12C, which is hook-shaped or bridge-shaped and preferably positioned above said first layer 10.

In a preferred embodiment, said at least one cavity 11 comprises a plurality of cavities 11 adapted to house at least one first portion 12A of a plurality of electrodes 12; preferably, the cavities 11 of said plurality of cavities 11 are arranged in matrix form, the centre of each cavity 11 being in particular positioned at a distance comprised between 2.5 mm and 70 mm, preferably at a distance of approximately 10 mm, from the centre of a contiguous cavity 11.

Moreover, said cavity 11 is adapted to house a conductive paste (not shown in the drawings), in particular water-based, which allows creating an optimal connection between the skin of a living being and said electrode 12, in particular said first portion 12P of the electrode 12.

Preferably, said conductive paste comprises the following components: Polyoxyethylene (20) Cetyl Ether, Water, Glycerin, Calcium Carbonate, 1,2-Propanediol, Potassium Chloride, Gelwhite®, Sodium Chloride, Polyoxyethylene (20) Sorbitol, Methylparaben, Propylparaben.

In particular, said conductive paste is the product known as Ten 20® Conductive Paste, available from Weaver and Company.

In accordance with the present invention, said device 1 further comprises a second layer 20 consisting of cross-linked polymers having a higher density than the first layer 10, said second layer 20 being adapted to give mechanical consistency to the device 1, to incorporate at least one second portion 12B of said electrode 12, and to allow it to be connected to a printed circuit board 2.

In particular, also the second layer 20 is transparent to ultrasounds and allows obtaining an acoustic impedance adaptation such that a joint acquisition of said at least one surface electromyographic signal and of said at least one echographic image of a same muscle portion can be obtained.

Preferably, said second layer 20 is made of rubber, in particular said second layer 20 having a base mass/catalyst concentration (or mixing ratio) substantially equivalent to a ratio of 1 to 1.

In substance, said second layer 20 has a base mass/catalyst concentration substantially suitable for satisfying the following relation (B):

$$base\ mass : catalyst = 1 : 1$$

Preferably, also for the second layer 20 said base mass and said catalyst consist of the material known as Elite Double 8®, available from company Zhermack S.p.A.

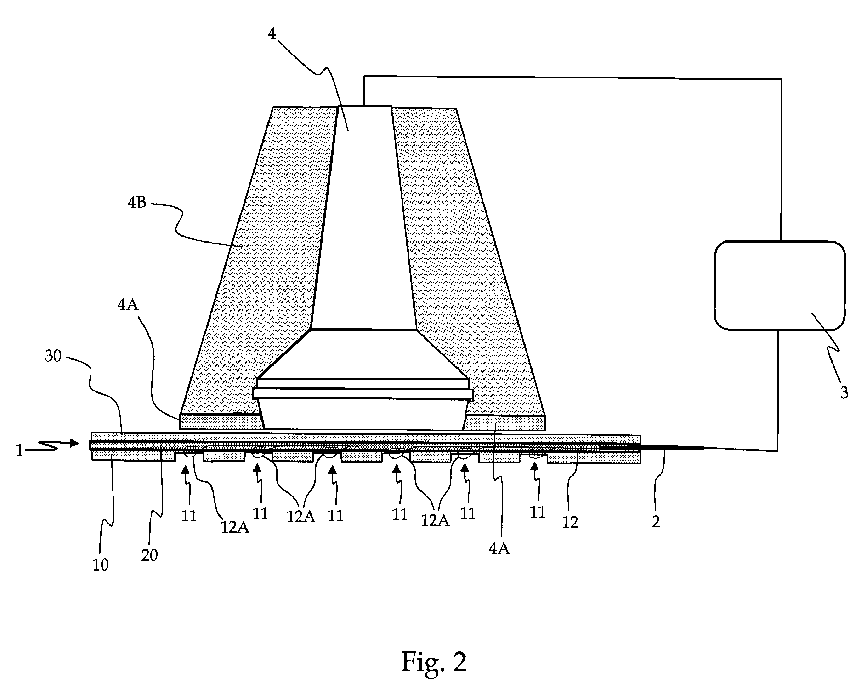

Fig. 2 shows a sectional view of a second embodiment of the device 1 according to the present invention.

In this figure, one can see that said device 1 comprises a third layer 30 for facilitating the coupling of the device 1 to an echographic probe 4, in particular said third layer 30 being made of a material substantially corresponding to that of the first layer 10.

As a consequence, also the third layer 30 consists of cross-linked polymers; preferably, said third layer 30 is made of rubber, in particular said third layer 30 having a base mass/catalyst concentration (or mixing ratio) substantially equivalent to a ratio of 1 to 3.

In this embodiment, also the echographic probe 4 preferably comprises at least one first surface 4A suitable for being coupled to said third layer 30, in particular said at least one surface 4A being made of a material substantially corresponding to that of the third layer 30.

The echographic probe 4 preferably further comprises at least one second surface 4B for supporting and/or holding up said echographic probe 4.

In Fig. 2 it can then be observed that also the echographic probe 4 is connected to said processing and/or display system 3, so that a joint acquisition of said at least one surface electromyographic signal and of at least one echographic image of a same portion of said muscle can be obtained.

It must be pointed out that the representation shown in Fig. 2 of the echographic probe 4 and of the connection thereof to the processing and display system 3 may also be adopted in the first embodiment of the device 1 shown in Figs. 1a and 1b; for example, the echographic probe 4 of Fig. 2 may differ from an echographic probe (not shown) suitable for the first embodiment for lacking the first surface 4A. It is also clear that said echographic probe 4 may have a different shape than shown in Fig. 2.

It is apparent from the above description that the particular design of the device 1 according to the present invention allows to overcome the drawbacks of the prior art and to provide a device 1 which is suitable for acquiring at least one surface electromyographic signal (sEMG) in conjunction with at least one echographic image of a same portion of a muscle of a living being.

In fact, the special design of the device 1 allows positioning an echographic probe (e.g. like the one designated by reference numeral 4 in Fig. 2) over the electrodes 12, so that it will not be adversely affected by the presence of said electrodes 12 or from other parts making up the device 1, particularly the first layer 10 and possibly also the second layer 20 and the third layer 30.

In particular, the device 1 according to the present invention is so designed as to allow obtaining an echographic image which will not be deformed by the acoustic impedance of the materials that compose the device 1 itself, in particular those of said electrodes 12, of the first layer 10, and possibly also of the second layer 20 and the third layer 30.

Furthermore, the special provisions of the device 1 according to the present invention allow to prevent any short circuits from occurring between the elements that make up the device 1; in particular, the presence of the cavities 11 (which are adapted to house the electrodes 12 and a conductive paste) allows creating an optimal connection between the skin of a living being and said electrodes 12, while at the same time preventing any short circuits from occurring between the electrodes 12.

Thanks to the special provisions of the present invention, it is apparent that the device 1 is particularly suited to non-invasive applications.

The following will describe a method for manufacturing a device 1 for the joint acquisition of at least one surface electromyographic signal (sEMG) and one echographic image of a same portion of a muscle of a living being, in particular for non-invasive applications.

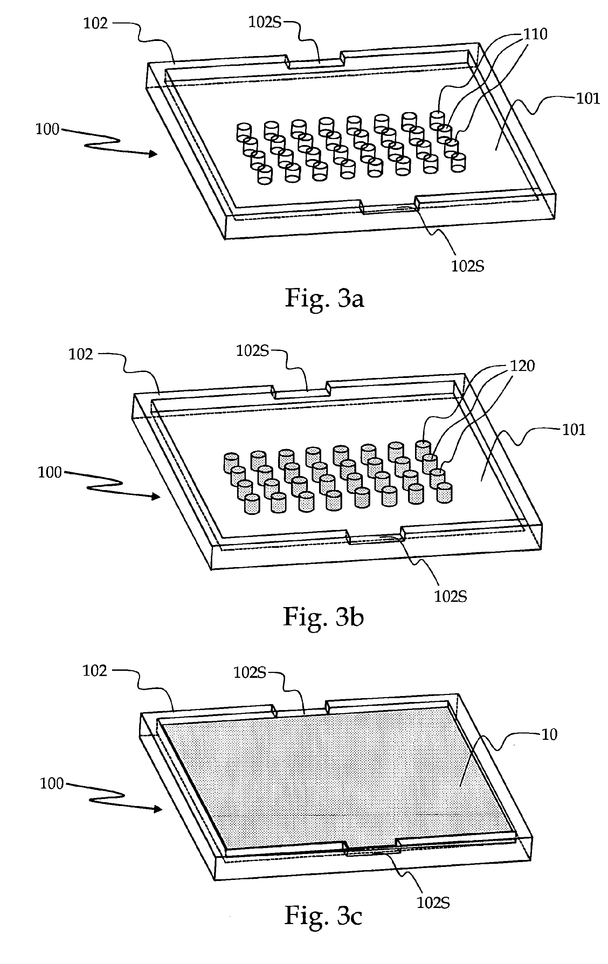

In particular, said method is shown in Figures 3a to 4d and comprises the following steps:

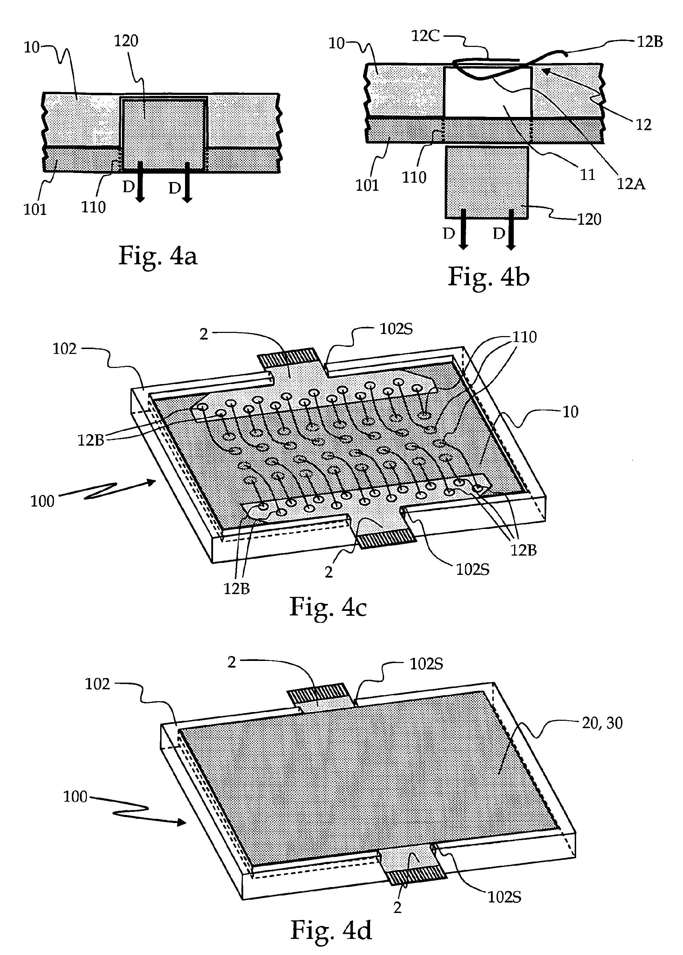

a) preparing a mould 100 having a base 101 and an edge 102 protruding from said base 101, said base 101 comprising at least one hole 110 (see Fig. 3a);
b) positioning at least one body 120, in particular cylindrical in shape, into said at least one hole 110 (see Fig. 3b);
c) inserting a first layer 10 consisting of cross-linked polymers into said mould 100 (see Fig. 3c), said first layer 10 being transparent to ultrasounds;
d) removing said at least one body 120 from said at least one hole 110 when the first layer 10 is polymerized, so as to obtain at least one cavity 11 in the first layer 10, in particular said removal of the body 120 from the hole 110 taking place in a direction (D in Figs. 4a and 4b) opposite to said first layer 10 (see Figs. 4a and 4b);
e) inserting into said cavity 11 at least one first portion 12A of an electrode 12 of the wire type, in particular made of metallic material, suitable for the acquisition of at least one surface electromyographic signal (sEMG), in particular said electrode 12 comprising a third portion 12C, which is hook-shaped or bridge-shaped and preferably positioned above said first layer 10 (see Fig. 4b).

Preferably, said wire electrode 12 is made of chlorinated silver; however, said electrode 12 may also be made of different materials.

Furthermore, the first layer 10 is so designed as to be adapted to adhere to the skin of said living being, so as to reduce the presence of any air bubbles.

The method for manufacturing the device 1 according to the present invention may further comprise the following steps:

f) positioning into the mould 100 at least one printed circuit board 2, in particular of the flexible type, and connecting a second portion 12B of the electrode 12

to said printed circuit board 2 (see Fig. 4c);
g) inserting into said mould 100 a second layer 20 consisting of cross-linked polymers having a higher density than the first layer 10, said second layer 20 being adapted to give mechanical consistency to the device 1, to incorporate at least one second portion 12B of said electrode 12, and to allow it to be connected to said printed circuit board 2 (see Fig. 4d).

According to the present invention, said step c) is preferably obtained by inserting into said mould 100 a first layer 10 made of rubber, in particular low-density rubber, said first layer 10 having a base mass/catalyst concentration (or mixing ratio) substantially equivalent to a ratio of 1 to 3, i.e. a base mass/catalyst concentration which can substantially satisfy the following relation (A):

$$base\ mass : catalyst = 1 : 3$$

Furthermore, according to the present invention, said step g) is obtained by inserting into said mould 100 a second rubber layer 20 having a base mass/catalyst concentration (or mixing ratio) substantially equivalent to a ratio of 1 to 1, i.e. a base mass/catalyst concentration which can substantially satisfy the following relation (B):

$$base\ mass : catalyst = 1 : 1$$

Said base mass and said catalyst of the first layer 10 and of the second layer 20 preferably consist of the material known as Elite Double 8®, available from company Zhermack S.p.A.
It is therefore clear that the first layer 10 and the second layer 20 are adapted to create an impedance adaptation such that a joint acquisition of said at least one surface electromyographic signal and at least one echographic image of a same portion of said muscle can be obtained.
The method according to the present invention may further comprise the step h) of inserting into said mould 100 a third layer 30, in particular made of a material substantially corresponding to that of the first layer 10.
Moreover, in accordance with the method of the present invention, said step a) may be carried out by making a plurality of holes 110 on said base 101, and said step b) may be carried out by positioning a plurality of bodies 120 into said plurality of holes 110.
Said step d) may then be carried out by inserting a portion of the printed circuit board 2 into at least one groove 102S (especially visible in Figures 3a to 3c and 4c to 4d) present in the edge 102.
In a preferred embodiment, said step e) is preceded by a step e-1) of making said electrode 12 of the wire type with a thickness comprised between 20 and

150 $\mu$m, in particular substantially corresponding to 50 $\mu$m.
Furthermore, said step e) and/or said step g) and/or said step h) may be followed by a step i) of inserting into said at least one cavity 11 a conductive paste, in particular water-based, which allows creating an optimal connection between the skin of a living being and said electrode 12.
The advantages of a device 1 for the acquisition of a surface electromyographic signal of a muscle of a living being, in particular for non-invasive applications, and of a related manufacturing method according to the present invention are apparent from the above description.
In particular, such advantages lie in the fact that the particular design of the device 1 allows to overcome the drawbacks of the prior art and to provide a device 1 which is suitable for acquiring at least one surface electromyographic signal (sEMG) in conjunction with at least one echographic image of a same portion of a muscle of a living being.
In fact, the special design of the device 1 allows positioning an echographic probe (designated by reference numeral 4 in Fig. 2, although said echographic probe may be made differently than shown in said Figure) over the electrodes 12, so that it will not be adversely affected by the presence of said electrodes 12 or from other parts making up the device 1, particularly the first layer 10, the second layer 20, and possibly also the third layer 30.
In particular, the device 1 according to the present invention is so designed as to allow obtaining an echographic image which will not be deformed by the acoustic impedance of the materials of the device 1 itself, in particular of said electrodes 12.
Furthermore, the special provisions of the device 1 according to the present invention allow to prevent any short circuits from occurring between the elements that make up the device 1; in particular, the presence of the cavities 11 (which are adapted to house the electrodes 12 and a conductive paste) allows creating an optimal connection between the skin of a living being and said electrodes 12, while at the same time preventing any short circuits from occurring between the electrodes 12.
Thanks to the special provisions of the present invention, it is apparent that the device 1 is particularly suited to non-invasive applications.
The device and method described herein by way of example may be subject to many possible variations without departing from the novelty spirit of the inventive idea; it is also clear that in the practical implementation of the invention the illustrated details may have different shapes or be replaced with other technically equivalent elements.
It can therefore be easily understood that the present invention is not limited to the above-described device and method, but may be subject to many modifications, improvements or replacements of equivalent parts and elements without departing from the inventive idea, as clearly specified in the following claims.

## Claims

1. A device (1) for the joint acquisition of at least one surface electromyographic signal and one echographic image of a same portion of a muscle of a living being, in particular for non-invasive applications, wherein said device (1) comprises, an electrode (12) for acquiring said at least one surface electromyographic signal and a first layer (10) consisting of cross-linked polymers, said first layer (10):

   - comprising a cavity (11) adapted to house a first portion (12A) of said electrode; and
   - being transparent to ultrasounds, so as to allow obtaining an acoustic impedance adaptation such that said joint acquisition of said at least one surface electromyographic signal and of said at least one echographic image of a same portion of said muscle can be obtained,

   wherein said electrode (12) is of the wire type and comprises a third portion (12C), which is hook-shaped or bridge-shaped and preferably positioned above said first layer (10).

2. A device (1) according to claim 1, **characterized in that** said first layer (10) consisting of cross-linked polymers is made of rubber, in particular low-density rubber, and has a base mass/catalyst concentration substantially equivalent to a ratio of 1 to 3.

3. A device (1) according to one or more of the preceding claims, **characterized in that** it comprises a second layer (20) consisting of cross-linked polymers having a higher density than the first layer (10), said second layer (20) being adapted to give mechanical consistency to the device (1), to incorporate at least one second portion (12B) of said electrode (12), and to allow it to be connected to a printed circuit board (2).

4. A device (1) according to one or more of the preceding claims, **characterized in that** said second layer (20) is transparent to ultrasounds and allows obtaining an acoustic impedance adaptation such that a joint acquisition of said at least one surface electromyographic signal and of said at least one echographic image of a same muscle portion can be obtained.

5. A device (1) according to one or more of the preceding claims 3 and 4, **characterized in that** said second layer (20) is made of rubber, in particular said second layer (20) having a base mass/catalyst concentration substantially equivalent to a ratio of 1 to 1.

6. A device (1) according to claim 1, **characterized in that** the thickness of said wire electrode (12) is comprised between 20 $\mu$m and 150 $\mu$m, in particular substantially equal to 50 $\mu$m.

7. A device (1) according to one or more of the preceding claims 1 and 6, **characterized in that** said electrode (12) comprises a second portion (12B) adapted to be connected to a printed circuit board (2), said printed circuit board (2) allowing said electrode (12) to be connected to a processing and/or display system (3).

8. A device (1) according to one or more of the preceding claims 1, 6 and 7, **characterized in that** said electrode (12) is made of chlorinated silver.

9. A device (1) according to one or more of the preceding claims, **characterized in that** said at least one cavity (11) comprises a plurality of cavities (11) adapted to house at least one first portion (12A) of a plurality of electrodes (12), in particular said cavities (11) being arranged in matrix form and being adapted to house a conductive paste, in particular water-based, which allows creating an optimal connection between the skin of a living being and said electrode (12), and in particular the centre of each cavity (11) being located at a distance comprised between 2.5 mm and 70 mm, preferably at a distance of approximately 10 mm, from the centre of a contiguous cavity (11).

10. A device (1) according to one or more of the preceding claims, **characterized in that** it comprises a third layer (30) for facilitating the coupling of the device (1) to an echographic probe (4), in particular said third layer (30) being made of a material substantially corresponding to that of the first layer (10).

11. A method for manufacturing a device (1) for the joint acquisition of at least one surface electromyographic signal and one echographic image of a same portion of a muscle of a living being, in particular for non-invasive applications, said method comprising the following steps:

   a) preparing a mould (100) having a base (101) and an edge (102) protruding from said base (101), said base (101) comprising a hole (110);
   b) positioning at body (120), in particular cylindrical in shape, into said hole (110);
   c) inserting a first layer (10) consisting of cross-linked polymers into said mould (100), said first layer (10) being transparent to ultrasounds;
   d) removing said body (120) from said hole (110) when the first layer (10) is polymerized, so as to obtain a cavity (11) in the first layer (10), in particular said removal of the body (120) from the hole (110) taking place in a direction (D) opposite to said first layer (10);

e) inserting into said cavity (11) a first portion (12A) of an electrode (12) of the wire type suitable for the acquisition of at least one surface electromyographic signal, said electrode (12) comprising a third portion (12C), which is hook-shaped or bridge-shaped and preferably positioned above said first layer (10).

12. A method (1) according to claim 11, **characterized in that** it further comprises the following steps:

f) positioning into the mould (100) at least one printed circuit board (2), in particular of the flexible type, and connecting a second portion (12B) of the electrode (12) to said printed circuit board (2);

g) inserting into said mould (100) a second layer (20) consisting of cross-linked polymers having a higher density than the first layer (10), said second layer (20) being adapted to give mechanical consistency to the device (1), to incorporate at least one second portion (12B) of said electrode (12), and to allow it to be connected to said printed circuit board (2), in particular said step g) being obtained by inserting into said mould (100) a second rubber layer (20), in particular having a base mass/catalyst concentration substantially equivalent to a ratio of 1 to 1;

h) inserting into said mould (100) a third layer (30), in particular made of a material substantially corresponding to that of the first layer (10);

i) inserting into said at least one cavity (10) a conductive paste, in particular a water-based conductive paste, which allows creating an optimal connection between the skin of a living being and said electrode (12).

13. A method (1) according to claim 11, **characterized in that** said step c) is obtained by inserting into said mould (100) a first layer (10) made of rubber, in particular low-density rubber, said first layer (10) having a base mass/catalyst concentration substantially equivalent to a ratio of 1 to 3, and **in that** said step d) is carried out by inserting a portion of the printed circuit board (2) into at least one groove (102S) present in the edge (102).

14. A method (1) according to claim 11, **characterized in that** said step e) is preceded by a step e-1) of making said electrode (12) of the wire type with a thickness comprised between 20 and 150 $\mu$m, in particular substantially corresponding to 50 $\mu$m.

15. A method (1) according to claim 11, **characterized in that** said step a) is carried out by making a plurality of holes (110) on said base (101), and said step b) is carried out by positioning a plurality of bodies (120) into said plurality of holes (110).

**Patentansprüche**

1. Gerät (1) zur gemeinsamen Erfassung von zumindest einem elektromyografischen Oberflächensignal und einem echografischen Bild eines selben Teils eines Muskels eines Lebewesens, insbesondere für nicht-invasive Anwendungen, wobei das Gerät (1) eine Elektrode (12) zum Erfassen des zumindest einen elektromyografischen Oberflächensignals und eine erste Schicht (10), die aus quervernetzten Polymeren besteht, umfasst, wobei die erste Schicht (10):

- eine Kavität (11) umfasst, die angepasst ist, einen ersten Teil (12A) der Elektrode aufzunehmen; und
- transparent ist für Ultraschall, um es zu ermöglichen, eine akustische Impedanzadaption zu erhalten, sodass die gemeinsame Erfassung von dem zumindest einen elektromyografischen Oberflächensignal und von dem zumindest einen echografischen Bild eines selben Teils des Muskels erhalten werden kann,

wobei die Elektrode (12) vom Drahttyp ist und einen dritten Teil (12C) umfasst, welcher eine Hakenform, oder eine Brückenform aufweist und bevorzugt über der ersten Schicht (10) positioniert ist.

2. Gerät (1) nach Anspruch 1, **dadurch gekennzeichnet, dass** die erste Schicht (10), die aus quervernetztem Polymeren besteht, aus Gummi, insbesondere Gummi geringer Dichte, gemacht ist, und eine Basismasse/Katalysator-Konzentration aufweist, die im Wesentlichen gleich einem Verhältnis von 1:3 ist.

3. Gerät (1) nach einem oder mehreren der vorangegangenen Ansprüche, **dadurch gekennzeichnet, dass** es eine zweite Schicht (20) umfasst, die aus quervernetzten Polymeren besteht, welche eine höhere Dichte aufweisen als die erste Schicht (10), wobei die zweite Schicht (20) angepasst ist, dem Gerät (1) mechanische Konsistenz zu verleihen, zumindest einen zweiten Teil (12B) der Elektrode (12) zu enthalten, und es dem Gerät zu ermöglichen, mit einer Leiterplatte (2) verbunden zu werden.

4. Gerät (1) nach einem oder mehreren der vorangegangenen Ansprüche, **dadurch gekennzeichnet, dass** die zweite Schicht (20) für Ultraschall transparent ist und es ermöglicht, eine akustische Impedanzadaption zu erhalten, sodass eine gemeinsame Erfassung von dem zumindest einem elektromyografischen Oberflächensignal und dem zumindest einen echografischen Bild eines selben Muskelteils erhalten werden kann.

5. Gerät (1) nach einem oder mehreren der vorangegangenen Ansprüche 3 und 4, **dadurch gekennzeichnet, dass** die zweite Schicht (20) aus Gummi gemacht ist, wobei die zweite Schicht (20) insbesondere eine Basismasse/Katalysator-Konzentration aufweist, die im Wesentlichen gleich einem Verhältnis von 1:1 ist.

6. Gerät (1) nach Anspruch 1, **dadurch gekennzeichnet, dass** die Dicke der Drahtelektrode (12) im Bereich zwischen 20 μm und 150 μm liegt, und insbesondere im Wesentlichen gleich 50 μm ist.

7. Gerät (1) nach einem oder mehreren der vorangegangenen Ansprüche 1 und 6, **dadurch gekennzeichnet, dass** die Elektrode (12) einen zweiten Teil (12B) umfasst, der angepasst ist, mit einer Leiterplatte (2) verbunden zu werden, wobei die Leiterplatte (2) es der Elektrode (12) ermöglicht, mit einem Verarbeitungs- und/oder Anzeigesystem (3) verbunden zu werden.

8. Gerät (1) nach einem oder mehreren der vorangegangenen Ansprüche 1, 6 und 7, **dadurch gekennzeichnet, dass** die Elektrode (12) aus chloriertem Silber gemacht ist.

9. Gerät (1) nach einem oder mehreren der vorangegangenen Ansprüche, **dadurch gekennzeichnet, dass** die zumindest eine Kavität (11) eine Mehrzahl von Kavitäten (11) umfasst, die angepasst sind, zumindest einen ersten Teil (12A) einer Mehrzahl von Elektroden (12) aufzunehmen, wobei die Kavitäten (11) insbesondere in Matrixform angeordnet sind und angepasst sind, eine leitende, insbesondere wasserbasierte, Paste aufzunehmen, was es ermöglicht, eine optimale Verbindung zwischen der Haut eines Lebewesens und der Elektrode (12) zu erzeugen, und wobei insbesondere das Zentrum jeder Kavität (11) in einem Abstand gelegen ist, der zwischen 2.5 mm und 70 mm, bevorzugt in einem Abstand von ungefähr 10 mm, vom Zentrum einer angrenzenden Kavität (11) liegt.

10. Gerät (1) nach einem oder mehreren der vorangegangenen Ansprüche, **dadurch gekennzeichnet, dass** es eine dritte Schicht (30) zum Erleichtern des Ankoppelns des Geräts (1) an eine echografische Sonde (4) umfasst, wobei die dritte Schicht (30) insbesondere aus einem Material gemacht ist, das im Wesentlichen zu dem der ersten Schicht (10) korrespondiert.

11. Verfahren zum Herstellen eines Geräts (1) zur gemeinsamen Erfassung von zumindest einem elektromyografischen Oberflächensignal und einem echografischen Bild eines selben Teils eines Muskels eines Lebewesens, insbesondere für nicht-invasive Anwendungen, wobei das Verfahren die folgenden Schritte umfasst:

    a) Vorbereiten einer Form (100), die eine Basis (101) und eine Kante (102), die von der Basis (101) vorsteht, aufweist, wobei die Basis (101) ein Loch (110) umfasst;
    b) Positionieren eines Körpers (120), insbesondere mit einer zylindrischen Form, in das Loch (110);
    c) Einbringen einer ersten Schicht (10), die aus quervernetzten Polymeren besteht, in die Form (100), wobei die erste Schicht (10) transparent für Ultraschall ist;
    d) Entfernen des Körpers (120) aus dem Loch (110), wenn die erste Schicht (10) polymerisiert ist, um so eine Kavität (11) in der ersten Schicht (10) zu erhalten, wobei das Entfernen des Körpers (120) aus dem Loch (110) insbesondere in einer Richtung (D) erfolgt, die entgegengesetzt zu der ersten Schicht (10) ist;
    e) Einbringen eines ersten Teils (12A) einer Elektrode (12) vom Drahttyp, die zur Erfassung von zumindest einem elektromyografischen Oberflächensignal geeignet ist, in die Kavität (11), wobei die Elektrode (12) einen dritten Teil (12C) umfasst, der hakenförmig oder brückenförmig ist und bevorzugt über der ersten Schicht (10) positioniert ist.

12. Verfahren (1) nach Anspruch 11, **dadurch gekennzeichnet, dass** es des Weiteren folgende Schritte umfasst:

    f) Positionieren zumindest einer Leiterplatte (2), insbesondere vom flexiblen Typ, in der Form (100) und Verbinden eines zweiten Teils (12B) der Elektrode (12) mit der Leiterplatte (2);
    g) Einbringen einer zweiten Schicht (20), die aus quervernetzten Polymeren besteht, welche eine höhere Dichte aufweisen als die erste Schicht (10), in die Form (100), wobei die zweite Schicht (20) angepasst ist, dem Gerät (1) mechanische Konsistenz zu verleihen, zumindest einen zweiten Teil (12B) der Elektrode (12) aufzunehmen, und es dem Gerät zu ermöglichen, mit der Leiterplatte (2) verbunden zu werden, wobei insbesondere der Schritt g) durch Einbringen einer zweiten Gummischicht (20), welche insbesondere eine Basismasse/Katalysator-Konzentration aufweist, die im Wesentlichen gleich einem Verhältnis von 1:1 ist, in die Form (100) erhalten wird;

h) Einbringen einer dritten Schicht (30), die insbesondere aus einem Material gemacht ist, das im Wesentlichen zu dem der ersten Schicht (10) korrespondiert, in die Form (100);

i) Einbringen einer leitenden Paste, insbesondere einer wasserbasierten leitenden Paste, die es ermöglicht, eine optimale Verbindung zwischen der Haut des Lebewesens und der Elektrode (12) zu erzeugen, in die zumindest eine Kavität (10).

**13.** Verfahren (1) nach Anspruch 11, **dadurch gekennzeichnet, dass** der Schritt c) durch Einbringen einer ersten Schicht (10), die aus Gummi, insbesondere Gummi geringer Dichte, gemacht ist, in die Form (100) erhalten wird, wobei die erste Schicht (10) eine Basismasse/Katalysator-Konzentration aufweist, die im Wesentlichen gleich einem Verhältnis von 1:3 ist, und dass der Schritt d) durch Einbringen eines Teils der Leiterplatte (2) in zumindest eine Nut (102S), die in der Kante (102) vorhanden ist, ausgeführt wird.

**14.** Verfahren (1) nach Anspruch 11, **dadurch gekennzeichnet, dass** dem Schritt e) ein Schritt e-1) des Herstellens der Elektrode (12) des Drahttyps mit einer Dicke, die zwischen 20 und 150 µm liegt und insbesondere im Wesentlichen 50 µm entspricht, vorausgeht.

**15.** Verfahren (1) nach Anspruch 11, **dadurch gekennzeichnet, dass** der Schritt a) durch Erzeugen einer Mehrzahl von Löchern (110) an der Basis (101) ausgeführt wird, und dass der Schritt b) durch Positionieren einer Mehrzahl von Körpern (120) in der Mehrzahl von Löchern (110) ausgeführt wird.

**Revendications**

**1.** Dispositif (1) d'acquisition conjointe d'au moins un signal électromyographique de surface et d'une image échographique d'une même partie d'un muscle d'un être vivant, destiné en particulier à des applications non-invasives, dans lequel ledit dispositif (1) comprend une électrode (12) destinée à acquérir ledit au moins un signal électromygraphique de surface et une première couche (10) constituée de polymères réticulés, ladite première couche (10) :

- comprenant une cavité (11) adaptée pour loger une première partie (12A) de ladite électrode ; et
- étant transparente aux ultrasons, de manière à pouvoir obtenir une adaptation d'impédance acoustique telle que ladite acquisition conjointe dudit au moins un signal électromyographique de surface et de ladite au moins une image échographique d'une même partie dudit muscle puisse être obtenue,

ladite électrode (12) étant du type filaire et comprenant une troisième partie (12C), qui est en forme de crochet ou de pont et qui est de préférence positionnée au-dessus de ladite première couche (10).

**2.** Dispositif (1) selon la revendication 1, **caractérisé en ce que** ladite première couche (10) constituée de polymères réticulés est réalisée en caoutchouc, notamment en caoutchouc à basse densité, et a une concentration catalyseur/masse de base sensiblement équivalente à un rapport de 1 à 3.

**3.** Dispositif (1) selon l'une ou plusieurs des revendications précédentes, **caractérisé en ce qu'**il comprend une deuxième couche (20) constituée de polymères réticulés ayant une densité supérieure à celle de la première couche (10), ladite deuxième couche (20) étant adaptée pour conférer une résistance mécanique au dispositif (1), pour incorporer au moins une deuxième partie (12B) de ladite électrode (12), et pour permettre à celle-ci d'être reliée à une carte de circuit imprimé (2).

**4.** Dispositif (1) selon l'une ou plusieurs des revendications précédentes, **caractérisé en ce que** ladite deuxième couche (20) est transparente aux ultrasons et permet d'obtenir une adaptation d'impédance acoustique de façon à ce que l'acquisition conjointe dudit au moins un signal électromyographique de surface et ladite au moins une image échographique d'une même partie de muscle puisse être obtenue.

**5.** Dispositif (1) selon l'une ou plusieurs des revendications précédentes 3 et 4, **caractérisé en ce que** ladite deuxième couche (20) est réalisée en caoutchouc, en particulier ladite deuxième couche (20) ayant une concentration catalyseur/masse de base sensiblement équivalente à un rapport de 1 à 1.

**6.** Dispositif (1) selon la revendication 1, **caractérisé en ce que** l'épaisseur de ladite électrode filaire (12) est comprise entre 20 µm et 150 µm, en particulier est sensiblement égale à 50 µm.

**7.** Dispositif (1) selon l'une ou plusieurs des revendications précédentes 1 à 6, **caractérisé en ce que** ladite électrode (12) comprend une deuxième partie (12B) adaptée pour être reliée à une carte à circuit imprimé (2), ladite carte de circuit imprimé (2) permettant de relier ladite électrode (12) à un système de traitement et/ou d'affichage (3).

**8.** Dispositif (1) selon l'une ou plusieurs des revendications précédentes 1, 6 et 7, **caractérisé en ce que** ladite électrode (12) est en argent chloré.

9.  Dispositif (1) selon l'une ou plusieurs des revendications précédentes, **caractérisé en ce que** ladite au moins une cavité (11) comprend une pluralité de cavités (11) adaptées pour loger au moins une première partie (12A) d'une pluralité des électrodes (12), en particulier lesdites cavités (11) étant disposées sous forme de matrice et étant adaptées pour loger une pâte conductrice, en particulier à base d'eau, qui permet d'établir une connexion optimale entre la peau d'un être vivant et ladite électrode (12), et en particulier le centre de chaque cavité (11) étant située à une distance comprise entre 2,5 mm et 70 mm, de préférence à une distance d'environ 10 mm, à partir du centre d'une cavité contiguë (11).

10. Dispositif (1) selon l'une ou plusieurs des revendications précédentes, **caractérisé en ce qu'**il comprend une troisième couche (30) destinée à faciliter le couplage du dispositif (1) à une sonde échographique (4), en particulier ladite troisième couche (30) étant constituée d'une matière correspondant sensiblement à celle de la première couche (10).

11. Procédé de fabrication d'un dispositif (1) destiné à l'acquisition conjointe d'au moins un signal électromyographique de surface et d'une image échographique d'une même partie d'un muscle d'un être vivant, destiné notamment à des applications non-invasives, ledit procédé comprenant les étapes suivantes :

    a) préparer un moule (100) comportant une base (101) et un bord (102) faisant saillie de ladite base (101), ladite base (101) comportant un trou (110) ;
    b) positionner un corps (120), en particulier de forme cylindrique, dans ledit trou (110) ;
    c) insérer une première couche (10) constituée de polymères réticulés dans ledit moule (100), ladite première couche (10) étant transparente aux ultrasons ;
    d) retirer ledit corps (120) dudit trou (110) lorsque la première couche (10) est polymérisée, de manière à obtenir une cavité (11) dans la première couche (10), en particulier ledit retrait du corps (120) dudit trou (110) se faisant dans une direction (D) opposée à ladite première couche (10) ;
    e) insérer dans ladite cavité (11) une partie (12A) d'une électrode (12) de type filaire adaptée à l'acquisition d'au moins un signal électromyographique de surface, ladite électrode (12) comprenant une troisième partie (12C), qui est en forme de crochet ou de pont et positionnée de préférence au-dessus de ladite première couche (10).

12. Procédé (1) selon la revendication 11, **caractérisé en ce qu'**il comprend en outre les étapes suivantes consistant à :

    f) positionner dans le moule (100) au moins une carte de circuit imprimé (2), en particulier du type souple, et relier une deuxième partie (12B) de l'électrode (12) à ladite carte de circuit imprimé (2) ;
    g) insérer dans ledit moule (100) une deuxième couche (20) constituée de polymères réticulés de densité supérieure à celle de la première couche (10), ladite deuxième couche (20) étant adaptée pour conférer une résistance mécanique au dispositif (1), pour incorporer au moins une deuxième partie (12B) de ladite électrode (12) et pour relier celle-ci à ladite carte de circuit imprimé (2), en particulier ladite étape g) étant obtenue par insertion dans ledit moule (100) d'une deuxième couche de caoutchouc (20), en particulier ayant une concentration catalyseur/masse de base sensiblement équivalente à un rapport de 1 à 1 ;
    h) insérer dans ledit moule (100) une troisième couche (30), en particulier constituée d'une matière correspondant sensiblement à celle de la première couche (10) ;
    i) insérer dans ladite au moins une cavité (10) d'une pâte conductrice, en particulier d'une pâte conductrice à base d'eau, qui permet d'établir une connexion optimale entre la peau d'un être vivant et ladite électrode (12).

13. Procédé (1) selon la revendication 11, **caractérisé en ce que** ladite étape c) est obtenue par l'insertion dans ledit moule (100) d'une première couche (10) en caoutchouc, notamment en caoutchouc à basse densité, ladite première couche (10) ayant une concentration en catalyseur/masse de base sensiblement équivalente à un rapport de 1 à 3, et **en ce que** ladite étape d) est réalisée par l'insertion d'une partie de la carte de circuit imprimé (2) dans au moins une gorge (102S) ménagée dans le bord (102).

14. Procédé (1) selon la revendication 11, **caractérisé en ce que** ladite étape e) est précédée d'une étape e-1) consistant à réaliser ladite électrode (12) de type filaire avec une épaisseur comprise entre 20 et 150 $\mu$m, en particulier correspondant sensiblement à 50 $\mu$m.

15. Procédé (1) selon la revendication 11, **caractérisé en ce que** ladite étape a) est réaliser en faisant une pluralité de trous (110) sur ladite base (101), et ladite étape b) est réalisée en positionnant une pluralité de corps (120) dans ladite pluralité de trous (110).

Fig. 1a

Fig. 1b

Fig. 2

EP 2 872 038 B1

Fig. 3a

Fig. 3b

Fig. 3c

Fig. 4a

Fig. 4b

Fig. 4c

Fig. 4d

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009089280 A

- WO 9208407 A

### Non-patent literature cited in the description

- **DE OLIVEIRA L.F. ; MENEGALDO L.L.** Individual-specific muscle maximum force estimation using ultrasound for ankle joint torque prediction using an EMG-driven Hill-type model. *J. Biomech.,* 19 October 2010, vol. 43 (14), 2816-21
- **RUDROFF T. ; STAUDENMANN D. ; ENOKA R. M.** Electromyographic measures of muscle activation and changes in muscle architecture of human elbow flexors during fatiguing contractions. *J. Appl. Physiol.,* June 2008, vol. 104 (6), 1720-6

- **MADEMLI L ; ARAMPATZIS A.** Mechanical and morphological properties of the triceps surae muscle-tendon unit in old and young adults and their interaction with a submaximal fatiguing contraction. *J Electromyogr. Kinesiol.,* February 2008, vol. 18 (1), 89-98
- **LORAM ID ; MAGANARIS CN ; LAKIE M.** Human postural sway results from frequent, ballistic bias impulses by soleus and gastrocnemius. *J. Physiol,* 01 April 2005, vol. 564, 295-311
- **HODGES PW ; PENGEL LH ; HERBERT RD ; GANDEVIA SC.** Measurement of muscle contraction with ultrasound imaging. *Muscle Nerve.,* June 2003, vol. 27 (6), 682-92